# EUROPEAN PATENT SPECIFICATION

(11) **EP 3 620 286 B1**
(45) Date of publication and mention of the grant of the patent: **29.12.2021**
(21) Application number: 19382957.9
(22) Date of filing: 04.11.2019
(51) Int. Cl.: B29C 45/14, A42B 3/22, A43B 3/22, G02B 1/14, G02B 1/18, G02B 5/20, G02B 5/23, G02B 7/00, G02B 27/00, A61F 9/02, A42C 2/00, G02B 27/01

(54) **ARC-SHAPED PHOTO-CHROMIC VISOR AND PRODUCTION METHOD THEREOF**
BOGENFÖRMIGES PHOTOCHROMES VISIER UND PRODUKTIONSMETHODE
VISIÈRE PHOTOCHROMIQUE EN FORME D'ARC ET MÉTHODE DE PRODUCTION

(43) Date of publication of application: 11.03.2020
(73) Proprietor: Mat Product & Technology, SL, 08223 Terrassa (ES)
(72) Inventor: MATEU CODINA, Xavier, 08230 Matadepera (ES); CADENS BALLARIN, Javier, 08830 Sant Boi de Llobregat (ES)
(74) Representative: Juncosa Miró, Jaime

(56) References cited:
- EP-A1- 2 407 041
- WO-A1-2019/145391
- WO-A2-2013/055217
- Anonymous: "Polycarbonate - Wikipedia", , 27 October 2020 (2020-10-27), XP55745389, Wikipedia Retrieved from the Internet: URL:https://en.wikipedia.org/wiki/Polycarb onate [retrieved on 2020-10-29]

## Description

### Technical field

The present invention is directed to an arc-shaped photo-chromic visor and to the production method thereof.

An arc-shaped photo-chromic visor is a screen intended to be placed in front of the face, typically integrated in a helmet or in google frame to protect the eyes of the wearer from impacts or incident light.

It will be understood that a photo-chromic visor is a visor which gets dark when direct sunlight impacts therein reducing its transparency to the visible light protecting the eyes from the incident light. Typically, the more sun light the photo-chromic material receives the darker it gets.

### State of the Art

It is known the use of photo-chromic materials integrated in visors or glasses.

The integration of said photo-chromic materials in an arc-shaped visor is also known for example through document EP2407041 which describes the integration of a photo-chromic foil in a recess placed outside of an arc-shaped visor, and alternatively in the inner side thereof.

Plastic materials are frequently non-transparent to the UV light, even when are transparent to the visible light. The photo-chromic foil reacts to the incident UV light getting darker and reducing its transparency to the visible light when receiving sun light including UV light, therefore on document EP2407041 said photo-chromic foil is placed where it receives direct incident sunlight, on the outside of the visor, because if placed inside the visor the plastic of said visor will prevent the UV light to reach the photo-chromic foil.

Document WO2013055217 describes a production method for an arc-shaped visor including a photo-chromic layer but including said layer in the outer surface thereof.

The photo-chromic foil is more sensible to scratches than the plastic constitutive of the rest of the visor. When the photo-chromic foil is placed outside the visor it suffers many accidental impacts and rubs which frequently produces scratches thereon. Document EP2407041 describes the protection of the photo-chromic foil with an integrated layer made of plastic.

Also, the capability of the photo-chromic foil to get darker is also affected by the temperature thereof. The photo-chromic foil suffers bigger thermal changes being placed on the outside of the visor, where no thermal protection is provided, also reaching higher temperatures due to direct solar radiation, this affecting adversely their functionality in extreme climate conditions.

Furthermore, the integration of the photo-chromic foil in the visor will produce anti-aesthetic lines on the peripheric edges of the photo-chromic foil and restrictions in the field of vision of the user if not covering all the visor.

A solution to those and other problems is provided by the proposed invention.

### Brief description of the invention

According to a first aspect, the present invention is directed to an arc-shaped photo-chromic visor production method.

Said method comprises the following steps, which are already known in the state of the art:
- open an arc-shaped visor mold by spacing apart at least the following mold main parts:
   ∘ a mold concave surface having a central portion;
   ∘ a mold convex central surface facing said central portion of the mold concave surface
- cut a flexible photo-chromic foil with a predefined shape and extending said cut flexible photo-chromic foil in contact with a surface of the arc-shaped visor mold;
- close the arc-shaped visor mold, inject melted plastic therein said plastic being selected to be transparent to visible light once hardened, and
- hardening the transparent plastic obtaining the arc-shaped photo-chromic visor including a photo-chromic foil.

It will be understood that a flexible photo-chromic foil is a layer made of a material which gets dark and less transparent to visible light when receives incident light in a certain predefined spectrum, typically UV light.

The present invention further comprises the following steps which are not known in the state of the art.

The photo-chromic foil is selected to be reactive under UV light in a portion of the UV-A range adjacent to the visible light range; the plastic to be injected in the arc-shaped visor mold is selected to be partially UV-transparent in the UV-A range adjacent to the visible light range once hardened; and during the extension step the photo-chromic foil is extended in contact with the mold convex central surface.

According to this proposed invention the arc-shaped photo-chromic visor obtained from this method includes a photo-chromic foil placed in the inner side of the arc-shaped photo-chromic visor, so that when said visor is integrated in a helmet, the incident light passes through the partially UV-transparent plastic previous to reach the photo-chromic foil.

Most plastics used to produce said arc-shaped visors are transparent to the visible light but opaque or almost totally opaque to the UV-light in most of the UV wavelength range. The photo-chromic foil reacts to the UV light. When said photo-chromic foil is placed on the inner side of the arc-shaped visor (the concave side) the plastic constitutive of the rest of the arc-shaped visor blocks the UV-light preventing the reaction of the photo-chromic foil.

According to the present invention the injected plastic is selected to be partially UV-transparent in the UV-A range adjacent to the visible light once hardened, i.e. that said plastic in not opaque to the UV-light in certain wavelengths adjacent to the visible light.

The photo-chromic foil is selected to be reactive to said wavelengths to which the injected plastic is partially transparent adjacent to the visible light, permitting the darkening of the photo-chromic foil despite being placed behind a plastic which can be opaque to some UV wavelengths.

It will be understood that the UV-A range is comprised between 315 and 400 nm.

According to an additional embodiment, in the injecting step the plastic is injected between the photo-chromic foil and the mold concave surface of the arc-shaped visor mold to produce an arc-shaped photo-chromic visor including an outer layer made of said partially UV-transparent plastic and an inner layer made of the photo-chromic foil placed in the concave side of the outer layer.

It will be understood that the outer side of the arc-shaped photo-chromic visor is the convex side and the inner side is the concave side.

The step of opening arc-shaped visor mold can further comprise spacing apart two inner lateral surfaces of said arc-shaped visor mold placed on opposed ends of the mold convex central surface, each facing a lateral portion of the inner convex surface and including an articulation pin molding cavity, so that a partition is defined between each inner lateral surface and the mold convex central surface which will be visible on the arc-shaped photo-chromic visor produced. Said articulation pin molding cavity is provided to generate an articulation pin on opposed facing ends of the inner side of the arc-shaped photo-chromic visor provided to allow the articulation of said arc-shaped photo-chromic visor in regard to a helmet.

In the cutting step the photo-chromic foil can be cut with the size and shape of the mold convex central surface and can be applied on said inner convex surface placing delimiting edges of said photo-chromic foil coincident with said partitions.

The photo-chromic foil cannot be placed covering the articulation pin molding cavity because it will prevent the insertion of the injected plastic therein, therefore the photo-chromic foil cannot cover all the surface of the arch-shaped photo-chromic visor to be produced. It is proposed to make some of the delimiting edges of the photo-chromic foil coincident with the partition cited above.

According to an additional embodiment, during the applying step the photo-chromic foil can be applied on the mold convex central surface with corresponding delimiting edges placed coincident with an upper arched edge and a lower arched edge of the arc-shaped photo-chromic visor to be produced, i.e. coincident with an upper arched edge and a lower arched edge of the mold convex central surface.

Preferably the photo-chromic foil is adhered to the mold convex central surface by static electricity previous to the closure of the arc-shaped visor mold. Said photo-chromic foil can be automatically placed on the mold convex central surface by an automatic deposition device.

According to the preferred embodiment, the photo-chromic foil is die-cut.

It is also proposed that the partially UV-transparent plastic is injected in the arc-shaped visor mold through injection ports placed between the mold convex central surface and the mold concave surface.

After the extraction of the photo-chromic visor from the arc-shaped visor mold, an anti-scratch coating and/or an anti-fog coating can be applied on the convex surface and/or on the concave surface of the arc-shaped photo-chromic visor, said coating being applied by a flow coating or a deep coating application method.

The flow coating application method consist on pouring liquid coating on the surface to be treated, said liquid coating flowing on the surface covering it, and thereafter drying or curing of the remaining liquid coating.

The deep coating application method consist on the immersion of the piece to be coated on liquid coating, its extraction and the drying or curing of the remaining liquid coating.

It is also proposed that, during the cutting step, a photo-chromic foil which includes an anti-scratch and/or an anti-fog coating or which has anti-scratch and/or anti-fog properties can be selected and cut.

According to a second aspect of the present invention, it relates to an arc-shaped photo-chromic visor obtained according to the method described in the preceding description.

Said arc-shaped photo-chromic visor includes an outer layer made of a plastic transparent to visible light and partially UV-transparent in the UV-A range adjacent to the visible light and an inner layer made of a photo-chromic foil reactive under UV light in a portion of the UV-A range adjacent to the visible light which is placed in the concave side of the outer layer.

The arc-shaped photo-chromic visor can further comprise two articulation pins on two facing opposed ends of the concave surface thereof, and two partitions, each partition surrounding the articulation pin or connecting an upper arched edge with a lower arched edge of the arc-shaped photo-chromic visor, and wherein the photo-chromic foil includes delimiting edges coincident with said partitions.

The thickness of the outer layer is preferably comprised between 1mm and 4 mm.

The partially UV-transparent plastic is made of a material selected among Polycarbonate also named PC, Polymethilmetacrilate also named PMMA, ADC allyl diglycol carbonate also named CR-39.

Other features of the invention appear from the following detailed description of an embodiment.

### Brief description of the Figures

The foregoing and other advantages and features will be more fully understood from the following detailed description of an embodiment with reference to the accompanying drawings, to be taken in an illustrative and non-limitative manner, in which:
Fig. 1 shows a flattened view of the arc-shaped photo-chromic visor according to a first embodiment;
Fig. 2 shows an exploded view of the arc-shaped photo-chromic visor shown on Fig. 1;
Fig. 3 shows a plan view of the arc-shaped visor mold in a closed position;
Fig. 4, shows a plan view of the arc-shaped visor mold shown in Fig. 3 in opened position after the production of the arc-shaped photo-chromatic visor.

### Detailed description of an embodiment

The foregoing and other advantages and features will be more fully understood from the following detailed description of an embodiment with reference to the accompanying drawings, to be taken in an illustrative and not limitative.

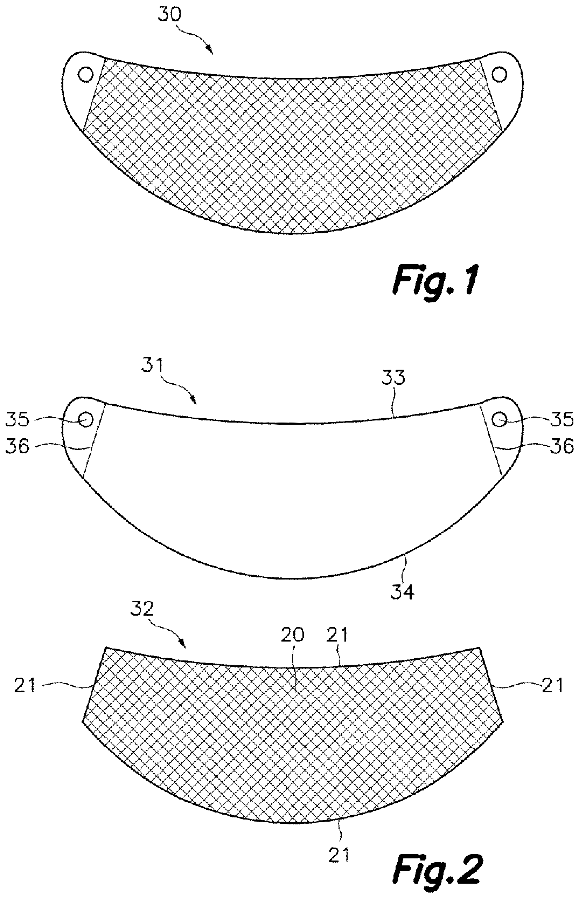

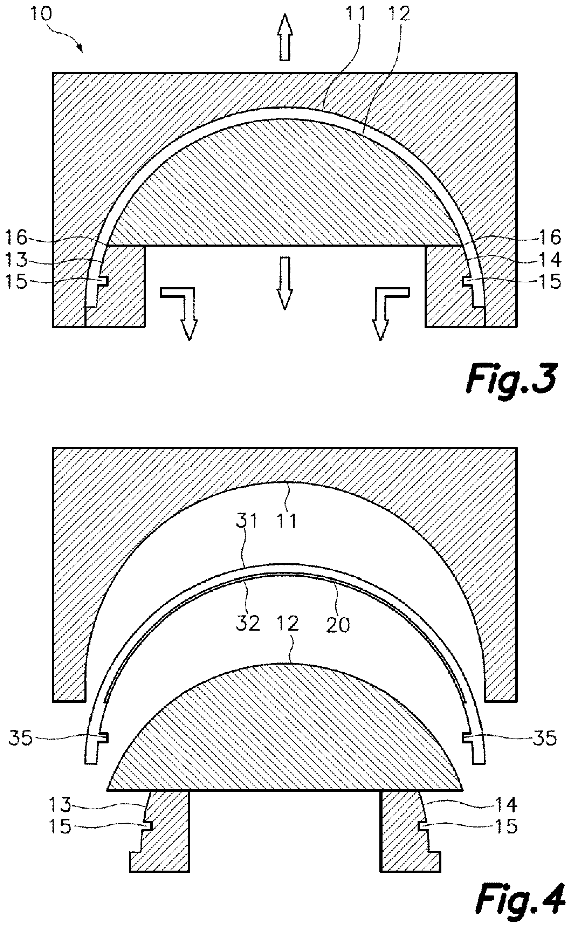

The present invention is directed to an arc-shaped photo-chromatic visor 30, shown on Figs. 1, 2 and 4, intended to be attached to a helmet to protect part of the face of a wearer wearing such helmet while allowing the vision therethrough, and to the production method thereof shown on Fig. 3 and 4.

Said proposed arc-shaped photo-chromatic visor 30 has photo-chromatic properties, i.e. that at least a part of it gets dark when an intense light reaches it, filtering part of said intense light.

The proposed arc-shaped photo-chromatic visor 30 comprises an outer layer 31, shown on the upper drawing of the Fig. 2, made of a plastic transparent to visible light and partially UV-transparent in the UV-A range adjacent to the visible light and an inner layer 32, shown on the lower drawing of the Fig. 2, which is made of a photo-chromic foil 20 reactive under UV light in a portion of the UV-A range adjacent to the visible light.

The inner layer 32 is placed in the concave side of the outer layer 31, as shown on Fig. 4. Said inner layer 32 is made by a photo-chromatic foil 20 which gets dark when affected by UV-A light in a range adjacent to the visible light range and is placed behind a plastic which is partially transparent to said particular UV-A light spectrum, allowing the darkening of the photo-chromatic foil despite being placed behind a plastic which can be opaque or mostly opaque to most of the UV light spectrum.

This position of the photo-chromatic foil 20 protects said foil from undesired scratches and from ambient conditions.

Said arc-shaped photo-chromatic visor 30 is produced in an arc-shaped visor mold 10 which comprises a mold concave surface 11 which central part faces a mold convex central surface 12 when closed, said two surfaces being spaced apart by a constant distance.

According to a particular embodiment the arc-shaped visor mold 10 further comprises two inner lateral surfaces 13 and 14 of said arc-shaped visor mold 10 placed on opposed ends of the mold convex central surface 12, each facing a lateral portion of the inner convex surface 11 and including an articulation pin molding cavity 15.

Said articulation pin mold cavities 15 create articulation pins 35 on opposed facing ends the inner side of the produced arc-shaped photo-chromic visor 30. Said articulation pins 35 can be used to articulate said visor to a helmet.

Between each inner lateral surfaces 13 and 14 and the mold convex central surface 12 a partition 16 is defined.

The proposed production method comprises cutting the photo-chromic foil 20 with a predefined shape, defining delimiting edges 21 of said photo-chromic foil 20, and extending said cut photo-chromic foil 20 in contact with the mold convex central surface 12 of the opened arc-shaped visor mold 10, said photo-chromic foil 20 being adhered to said surface for example by static electricity.

After that extension step the mold is closed and the outer layer 31 is overmolded on the photo-chromic foil 20, which define the inner layer 32, by the injection of a melted plastic in the closed arc-shaped visor mold 10, preferably through injection ports placed between the mold concave surface and the mold convex central surface.

According to a preferred embodiment the photo-chromic foil 20 is cut with the size and shape of the mold convex central surface 12 and is extended positioning the delimiting edges 21of the photo-chromic foil 20 coincident with the partitions 16 of the arc-shaped visor mold 10 and with the upper and lower arched edges 33 and 34 of the arc-shaped photo-chromic visor 30 to be produced, coincident with upper and lower edges of the mold convex central surface 12.

Thanks to this feature some of the delimiting edges 21 of the photo-chromic foil 20 coincides with the partitions 36 visible on the arc-shaped photo-chromic visor 30 produced.

According to the embodiment shown on Fig. 1 and 2 said two inner lateral surfaces 13 and 14 of the arc-shaped visor mold 10 completely defines end portions of the arc-shaped photo-chromic visor 30, one on each side of the zone defined by the mold convex central surface, defining partitions 36 transversal to the visor which connects the upper and lower arched edges 33, 34.

According to an alternative embodiment each of said two inner lateral surfaces 13 and 14 of the arc-shaped visor mold 10 is surrounded by the mold convex central surface 12, defining circular partitions 36 surrounding the articulation pins 35.

It will be understood that various parts of one embodiment of the invention can be freely combined with parts described in other embodiments, even being said combination not explicitly described, provided there is no harm in such combination.

## Claims

1. An arc-shaped photo-chromic visor production method, comprising the following steps:
opening an arc-shaped visor mold (10) by spacing apart at least the following mold parts:
• a mold concave surface (11) having a central portion;
• a mold convex central surface (12) facing said central portion of the mold concave surface (11);
cutting a flexible photo-chromic foil (20) with a predefined shape, said photo-chromic foil (20) being reactive under UV light in the UV-A range;
extending said cut flexible photo-chromic foil (20) in contact with the mold convex central surface (12) of the arc-shaped visor mold (10);
closing the arc-shaped visor mold (10),
injecting melted plastic therein said plastic being selected to be transparent to visible light once hardened, and
hardening the transparent plastic obtaining the arc-shaped photo-chromic visor (30) including a photo-chromic foil (20);
**characterized in that**
the photo-chromic foil (20) consist of a layer made of a material selected to be reactive under UV light in a portion of the UV-A range adjacent to the visible light range, the plastic being over molded on said layer constitutive of the photo-chromic foil (20);
the plastic to be injected in the arc-shaped visor mold (10) is selected to be partially UV-transparent in the UV-A range adjacent to the visible light once hardened but opaque or mostly opaque to most of the UV light spectrum

2. The arc-shaped photo-chromic visor production method according to claim 1 wherein during the injection step the partially UV-transparent plastic is injected between the photo-chromic foil (20) and the mold concave surface (11) of the arc-shaped visor mold (10).

3. The arc-shaped photo-chromic visor production method according to claim 1 or 2 wherein the step of opening arc-shaped visor mold (10) further comprises spacing apart two inner lateral surfaces (13, 14) of said arc-shaped visor mold (10) placed on opposed ends of the mold convex central surface (12), each facing a lateral portion of the inner convex surface (11) and including an articulation pin molding cavity (15), so that a partition (16) is defined between each inner lateral surface (13, 14) and the mold convex central surface (12); and wherein
in the cutting step the photo-chromic foil (20) is cut with the size and shape of the mold convex central surface (12) and is extended in contact with said mold convex central surface (12) placing delimiting edges (21) of said photo-chromic foil (20) coincident with said partitions (16).

4. The arc-shaped photo-chromic visor production method according to claim 3 wherein during the extension step the photo-chromic foil (20) is extended in contact with the mold convex central surface (12) with corresponding delimiting edges (21) placed coincident with an upper arched edge (33) and a lower arched edge (34) of the arc-shaped photo-chromic visor (30) to be produced.

5. The arc-shaped photo-chromic visor production method according to any preceding claim wherein the photo-chromic foil (20) is adhered to the mold convex central surface (12) by static electricity.

6. The arc-shaped photo-chromic visor production method according to any preceding claim wherein the photo-chromic foil (20) is automatically placed on the mold convex central surface by an automatic deposition device.

7. The arc-shaped photo-chromic visor production method according to any preceding claim wherein the partially UV-transparent plastic is injected in the arc-shaped visor mold (10) through injection ports placed between the mold convex central surface (12) and the mold concave surface (11).

8. The arc-shaped photo-chromic visor production method according to any preceding claim wherein the photo-chromic foil (20) is die-cut.

9. The arc-shaped photo-chromic visor production method according to any preceding claim wherein it further comprises, after the extraction of the arc-shaped photo-chromic visor (30) from the arc-shaped visor mold (10), the application of an anti-scratch coating and/or an anti-fog coating on the convex surface and/or on the concave surface of the photo-chromic visor applied by a flow coating or a deep coating application method.

10. An arc-shaped photo-chromic visor obtained according to the method described in the preceding claims, wherein said arc-shaped photo-chromic visor (30) consists of an outer layer (31) made of a plastic transparent to visible light and partially UV-transparent in the UV-A range but opaque or mostly opaque to most of the UV light spectrum, and an inner layer (32) reactive under UV light in a portion of the UV-A range placed in the concave side of the outer layer (31);
**characterized in that**
the inner layer (32) consists of a layer made of a material selected to be reactive under UV light in a portion of the UV-A range adjacent to the visible light wavelength range , and optionally with an anti-scratch coating and/or an anti-fog coating in the concave surface thereof, said layer constituting a photo-chromic foil (20).

11. The arc-shaped photo-chromic visor according to claim 10 wherein the arc-shaped photo-chromic visor (30) further comprises two articulation pins (35) on two facing opposed ends of the concave surface thereof, and two partitions (36) each partition surrounding one articulation pin (35) or connecting an upper arched edge (33) with a lower arched edge (34) of the arc-shaped photo-chromic visor (30), and wherein the photo-chromic foil (20) includes delimiting edges (21) coincident with said partitions (36).

12. The arc-shaped photo-chromic visor according to claim 10 or 11 wherein the thickness of the outer layer (31) is comprised between 1 and 4 mm.

13. The arc-shaped photo-chromic visor according to any preceding claim 10 to 12 wherein the partially UV-transparent plastic is made of a material selected among Polycarbonate, Polymethilmetacrilate and ADC, allyl diglycol carbonate.

## Patentansprüche

1. Verfahren zur Herstellung eines bogenförmigen photochromen Visiers, das die folgenden Schritte umfasst: Öffnen einer Form für das bogenförmige Visier (10) durch Beabstanden mindestens der folgenden Formteile:
• eine konkave Formfläche (11) mit einem zentralen Teil;
• eine konvexe zentrale Formfläche (12), die dem genannten zentralen Teil der konkaven Formfläche (11) gegenüberliegt;
Zuschneiden einer flexiblen photochromen Folie (20) in einer vorbestimmten Form, wobei die genannte photochrome Folie (20) unter UV-Licht im UV-A-Bereich reaktiv ist;
Ausbreiten der genannten zugeschnittenen flexiblen photochromen Folie (20) in Kontakt mit der konvexen zentralen Formfläche (12) der Form für das bogenförmige Visier (10);
Schließen der Form für das bogenförmige Visier (10),
Einspritzen von geschmolzenem Kunststoff in die Form, wobei der genannte Kunststoff so ausgewählt ist, dass er nach dem Aushärten für sichtbares Licht transparent ist, und
Aushärten des transparenten Kunststoffs unter Erzeugung des bogenförmigen photochromen Visiers (30) mit einer photochromen Folie (20);
**dadurch gekennzeichnet, dass**
die photochrome Folie (20) aus einer Schicht aus einem Material besteht, das so ausgewählt ist, dass es unter UV-Licht in dem an den Bereich des sichtbaren Lichts angrenzenden Segment des UV-A-Bereichs reaktiv ist, wobei der Kunststoff auf die genannte, die photochrome Folie (20) bildende Schicht aufgeformt wird;
der in die Form für das bogenförmige Visier (10) einzuspritzende Kunststoff so ausgewählt ist, dass er nach dem Aushärten in dem an das sichtbare Licht angrenzenden UV-A-Bereich teilweise UV-transparent, aber für den größten Teil des UV-Lichtspektrums undurchsichtig oder weitgehend undurchsichtig ist.

2. Verfahren zur Herstellung eines bogenförmigen photochromen Visiers nach Anspruch 1, bei dem während des Einspritzschritts der teilweise UV-transparente Kunststoff zwischen die photochrome Folie (20) und die konkave Formfläche (11) der Form für das bogenförmige Visier (10) eingespritzt wird.

3. Verfahren zur Herstellung eines bogenförmigen photochromen Visiers nach Anspruch 1 oder 2, wobei der Schritt des Öffnens der Form für das bogenförmige Visier (10) ferner das Beabstanden zweier innerer Seitenflächen (13, 14) der genannten Form für das bogenförmige Visier (10) umfasst, die an gegenüberliegenden Enden der konvexen zentralen Formfläche (12) angeordnet sind, jeweils einem seitlichen Abschnitt der konvexen inneren Formfläche (11) zugewandt sind und einen Formhohlraum für einen Gelenkbolzen (15) enthalten, sodass zwischen jeder inneren Seitenfläche (13, 14) und der konvexen zentralen Formfläche (12) eine Unterteilung (16) gebildet wird; und wobei
beim Schneideschritt die photochrome Folie (20) auf die Größe und Gestalt der konvexen zentralen Formfläche (12) zugeschnitten wird und in Kontakt mit der genannten konvexen zentralen Formfläche (12) ausgebreitet wird, wobei die Begrenzungskanten (21) der genannten photochromen Folie (20) mit den genannten Unterteilungen (16) zusammenfallend angeordnet werden.

4. Verfahren zur Herstellung eines bogenförmigen photochromen Visiers nach Anspruch 3, wobei während des Ausbreitschritts die photochrome Folie (20) in Kontakt mit der konvexen zentralen Formfläche (12) ausgebreitet wird, wobei die entsprechenden Begrenzungskanten (21) zusammenfallend mit einer oberen bogenförmigen Kante (33) und einer unteren bogenförmigen Kante (34) des herzustellenden bogenförmigen photochromen Visiers (30) angeordnet werden.

5. Verfahren zur Herstellung eines bogenförmigen photochromen Visiers nach einem der vorstehenden Ansprüche, wobei die photochrome Folie (20) durch statische Elektrizität an der konvexen zentralen Formfläche (12) anhaftet.

6. Verfahren zur Herstellung eines bogenförmigen photochromen Visiers nach einem der vorstehenden Ansprüche, bei dem die photochrome Folie (20) durch eine automatische Ablegevorrichtung automatisch auf die konvexe zentrale Formfläche gelegt wird.

7. Verfahren zur Herstellung eines bogenförmigen photochromen Visiers nach einem der vorstehenden Ansprüche, bei dem der teilweise UV-transparente Kunststoff durch zwischen der konvexen zentralen Fläche (12) und der konkaven Fläche (11) der Form angeordnete Einspritzöffnungen in die Form für das bogenförmige Visier (10) eingespritzt wird.

8. Verfahren zur Herstellung eines bogenförmigen photochromen Visiers nach einem der vorstehenden Ansprüche, wobei die photochrome Folie (20) gestanzt wird.

9. Verfahren zur Herstellung eines bogenförmigen photochromen Visiers nach einem der vorstehenden Ansprüche, umfassend ferner nach Entnahme des bogenförmigen photochromen Visiers (30) aus der Form (10) für das bogenförmige Visier das Aufbringen einer Anti-Kratz-Beschichtung und/oder einer Anti-Beschlag-Beschichtung auf die konvexe Oberfläche und/oder auf die konkave Oberfläche des photochromen Visiers durch ein Fließbeschichtungs- oder ein Tiefbeschichtungsverfahren.

10. Bogenförmiges photochromes Visier, erzeugt nach dem in den vorstehenden Ansprüchen beschriebenen Verfahren, wobei das genannte bogenförmige photochrome Visier (30) eine äußere Schicht (31) aus einem Kunststoff, der für sichtbares Licht transparent und im UV-A-Bereich teilweise UV-transparent, aber für den größten Teil des UV-Lichtspektrums undurchsichtig oder größtenteils undurchsichtig ist, und eine innere Schicht (32), die unter UV-Licht in einem Segment des UV-A-Bereichs reaktiv ist und auf der konkaven Seite der äußeren Schicht (31) angeordnet ist, umfasst;
**dadurch gekennzeichnet, dass**
die innere Schicht (32) aus einem Schichtmaterial besteht, das so ausgewählt ist, dass es unter UV-Licht in einem an den sichtbaren Lichtwellenlängenbereich angrenzenden Segment des UV-A-Bereichs reaktiv ist, und optional mit einer Anti-Kratz-Beschichtung und/oder einer Anti-Beschlag-Beschichtung auf seiner konkaven Oberfläche versehen ist, wobei die genannte Schicht eine photochrome Folie (20) bildet.

11. Bogenförmiges photochromes Visier nach Anspruch 10, wobei das bogenförmige photochrome Visier (30) ferner zwei Gelenkbolzen (35) an zwei einander gegenüberliegenden Enden seiner konkaven Oberfläche und zwei Unterteilungen (36) umfasst, wobei jede Unterteilung einen Gelenkbolzen (35) umgibt oder eine obere bogenförmige Kante (33) mit einer unteren bogenförmigen Kante (34) des bogenförmigen photochromen Visiers (30) verbindet, und wobei die photochrome Folie (20) mit den genannten Unterteilungen (36) zusammenfallende Begrenzungskanten (21) umfasst.

12. Bogenförmiges photochromes Visier nach Anspruch 10 oder 11, wobei die Dicke der äußeren Schicht (31) zwischen 1 und 4 mm liegt.

13. Bogenförmiges photochromes Visier nach einem der vorstehenden Ansprüche 10 bis 12, wobei der teilweise UV-transparente Kunststoff aus einem unter den Materialien Polycarbonat, Polymethylmethacrylat und ADC (Allyldiglykolcarbonat) ausgewählten Werkstoff besteht.

## Revendications

1. Une méthode de fabrication d'une visière photochromique en forme d'arc, comportant les étapes suivantes :
ouvrir un moule de visière en forme d'arc (10) en espaçant au moins les pièces de moule suivantes :
• une surface concave du moule (11) ayant une portion centrale ;
• une surface centrale convexe du moule (12) faisant face à cette portion centrale de la surface concave du moule (11) ;
découper une feuille photochromique flexible (20) avec une forme prédéfinie, cette feuille photochromique (20) étant réactive sous la lumière UV dans la gamme UV-A ;
étendre cette feuille photochromique flexible découpée (20) en contact avec la surface centrale convexe du moule (12) du moule de visière en forme d'arc (10) ;
fermer le moule de visière en forme d'arc (10)
injecter du plastique fondu en son intérieur, ce plastique étant sélectionné pour être transparent à la lumière visible dès qu'il a durci, et
durcir le plastique transparent en obtenant la visière photochromique en forme d'arc (30) comprenant une feuille photochromique (20) ;
**caractérisée en ce que**
la feuille photochromique (20) consiste en une couche faite en un matériau sélectionné pour être réactif sous la lumière UV dans une portion de la gamme UV-A adjacente à la gamme de lumière visible, le plastique étant surmoulée sur cette couche constituant la feuille photochromique (20) ;
le plastique à injecter dans le moule de visière en forme d'arc (10) est sélectionné pour être partiellement transparent dans la gamme UV-A adjacente à la lumière visible une fois durci mais opaque ou presque opaque à la plupart du spectre de lumière UV.

2. La méthode de fabrication d'une visière photochromique en forme d'arc, conformément à la revendication 1 où, durant l'étape d'injection, le plastique partiellement transparent au UV est injecté entre la feuille photochromique (20) et la surface concave du moule (11) du moule de visière en forme d'arc (10).

3. La méthode de fabrication d'une visière photochromique en forme d'arc, conformément à la revendication 1 où 2 où l'étape d'ouverture du moule de visière en forme d'arc (10) comporte par ailleurs espacer deux surfaces latérales intérieures (13, 14) de ce moule de visière en forme d'arc (10) placées sur des extrémités opposées de la surface centrale convexe du moule (12), chacune faisant face à une portion latérale de la surface convexe intérieure (11) et comprenant une cavité moulant une goupille d'articulation (15) de sorte que la cloison (16) est définie entre chaque surface latérale intérieure (13,14) et la surface centrale convexe de moule (12) ; et où
dans l'étape de découpage, la feuille photochromique (20) est découpée ayant la taille et la forme de la surface centrale convexe du moule (12) et s'étend en contact avec cette surface centrale convexe du moule (12) en plaçant des bords de délimitation (21) de cette feuille photochromique (20) coïncidant avec ces cloisons (16).

4. La méthode de fabrication d'une visière photochromique en forme d'arc conformément à la revendication 3, où durant l'étape d'extension, la feuille photochromique (20) est étendue en contact avec la surface centrale convexe du moule (12) avec les bords de délimitation correspondants (21) placés coïncidents avec un bord arqué supérieur (33) et un bord arqué inférieur (34) de la visière photochromique en forme d'arc (30) à fabriquer.

5. La méthode de fabrication d'une visière photochromique en forme d'arc, conformément à une quelconque des revendications précédentes, où la feuille photochromique (20) est adhérée à la surface centrale convexe du moule (12) par électricité statique.

6. La méthode de fabrication d'une visière photochromique en forme d'arc, conformément à une quelconque des revendications précédentes, où la feuille photochromique (20) est automatiquement placée sur la surface centrale convexe du moule par un dispositif de dépôt automatique.

7. La méthode de fabrication d'une visière photochromique en forme d'arc, conformément à une quelconque des revendications précédentes, où le plastique partiellement transparent au UV est injecté dans le moule de visière en forme d'arc (10) à travers des orifices d'injection placés entre la surface centrale convexe du moule (12) et la surface concave du moule (11).

8. La méthode de fabrication d'une visière photochromique en forme d'arc, conformément à une quelconque des revendications précédentes, où la feuille photochromique (20) est découpée à l'emporte-pièce.

9. La méthode de fabrication d'une visière photochromique en forme d'arc, conformément à une quelconque des revendications précédentes, où elle comporte par ailleurs , après l'extraction de la visière photochromique en forme d'arc (30) du moule de visière en forme d'arc (10) l'application d'un revêtement anti-rayures et/ou un revêtement antibuée sur la surface convexe et/ou sur la surface concave de la visière photochromique, appliqué par revêtement par aspersion ou une méthode d'application de revêtement profond.

10. Une visière photochromique en forme d'arc obtenue conformément à la méthode décrite dans les revendications précédentes, où cette visière photochromique en forme d'arc (30) consiste en une couche extérieure (31) faite en un plastique transparent à la lumière visible et partiellement transparent au UV dans la gamme UV A, mais opaque ou presque opaque à la plupart du spectre de lumière UV,
et une couche intérieure (32) réactive sous la lumière UV dans une portion de la gamme UV-A placée sur le côté concave de la couche extérieure (31) ;
**caractérisée en ce que**
la couche intérieure (32) consiste en une couche faite en un matériau sélectionné pour être réactif sous la lumière UV dans une portion de la gamme UV-A adjacente à la gamme de longueur d'ondes de lumière visible et, optionnellement, à un revêtement anti-rayure et/ou revêtement antibuée dans sa surface concave, cette couche constituant une feuille photochromique (20).

11. La visière photochromique en forme d'arc conformément à la revendication 10, où la visière photochromique en forme d'arc (30) comporte par ailleurs deux goupilles d'articulation (35) sur deux extrémités opposées se faisant face, de sa surface concave et deux cloisons (36), chaque cloison entourant une goupille d'articulation (35) ou reliant un bord arqué supérieur (33) avec un bord arqué inférieur (34) de la visière photochromique en forme d'arc (30) et où la feuille photochromique (20) comprend des bords de délimitation (21) coïncidant avec ces cloisons (36).

12. La visière photochromique en forme d'arc conformément à la revendication 10 ou 11, où l'épaisseur de la couche extérieure (31) est comprise entre 1 et 4 mm.

13. La visière photochromique en forme d'arc conformément à une quelconque des revendications précédentes 10 à 12, où le plastique partiellement transparent au UV est fait en un matériau sélectionné parmi le Polycarbonate, le Polyméthacrylate de méthyle et ADC, carbonate d'allyldiglycol.
